# EUROPÄISCHE PATENTSCHRIFT

(11) **EP 2 475 458 B1**
(45) Veröffentlichungstag und Bekanntmachung des Hinweises auf die Patenterteilung: **02.10.2013**
(21) Anmeldenummer: 10741985.5
(22) Anmeldetag: 12.08.2010
(51) Int. Cl.: B01J 31/30, C07C 2/32, B01J 31/26, C07C 2/36, B01J 31/24, B01J 31/14, B01J 31/02, C07C 11/02, C10G 50/00, B01J 31/22, B82Y 30/00

(54) **VERFAHREN ZUR OLIGOMERISIERUNG VON OLEFINEN**
METHOD FOR OLIGOMERISING OLEFINS
PROCÉDÉ D'OLIGOMÉRISATION D'OLÉFINES

(30) Priorität: 08.09.2009 DE 102009029284
(43) Veröffentlichungstag der Anmeldung: 18.07.2012
(73) Patentinhaber: Evonik Oxeno GmbH, 45772 Marl (DE)
(72) Erfinder: BÖING, Christian, 50679 Köln (DE); MASCHMEYER, Dietrich, 45657 Recklinghausen (DE); WINTERBERG, Markus, 45711 Datteln (DE); BUCHHOLZ, Stefan, 63456 Hanau (DE); MELCHER, Berthold, 91052 Erlangen (DE); HAUMANN, Marco, 91052 Erlangen (DE); WASSERSCHEID, Peter, 91054 Erlangen (DE)
(86) Internationale Anmeldenummer: PCT/EP2010/061770
(87) Internationale Veröffentlichungsnummer: WO 2011/029691

(56) Entgegenhaltungen:
- WO-A1-98/47616
- DE-A1-102006 019 460
- US-A1- 2003 181 775
- QINGMING FAN, YINGXIN LIU, FAN ZHENG, WEI YAN: "Preparation of Ni/SiO2 catalyst in ionic liquids for hydrogenation" FRONTIERS OF CHEMICAL ENGINEERING IN CHINA, Bd. 2(1), 19. Januar 2008 (2008-01-19), Seiten 63-68, XP009138431 ISSN: 1673-7369
- VIRTANEN P ET AL: "Towards one-pot synthesis of menthols from citral: Modifying Supported Ionic Liquid Catalysts (SILCAs) with Lewis and Bronsted acids" JOURNAL OF CATALYSIS, ACADEMIC PRESS, DULUTH, MN, US LNKD- DOI:10.1016/J.JCAT.2009.02.005, Bd. 263, Nr. 2, 25. April 2009 (2009-04-25), Seiten 209-219, XP026043758 ISSN: 0021-9517 [gefunden am 2009-03-10]

## Beschreibung

Die vorliegende Erfindung betrifft ein aus mehreren Komponenten aufgebautes Katalysatorsystem und dessen Verwendung in einem Verfahren zur Oligomerisierung von C₃- bis C₅-Olefinen aus olefinhaltigen Kohlenwasserstoffgemischen.

Die niedermolekularen Oligomere von Olefinen, insbesondere die Dimere von C₃-C₅-Olefinen, sind Zwischenprodukte, die beispielsweise für die Herstellung von Aldehyden, Carbonsäuren und Alkoholen eingesetzt werden. Die C₈-Olefine, die durch Oligomeriserung aus linearen Butenen entstehen, können durch Hydroformylierung und nachfolgender Hydrierung zu den entsprechenden Nonanolen umgesetzt werden, die wiederum vorwiegend für die Herstellung von Weichmachern Verwendung finden.

Für die Herstellung der Oligomere können als Ausgangsmaterialien reine Olefine mit Doppelbindung in α-Stellung, reine Olefine mit innenständiger Doppelbindung und Gemische dieser Olefine verwendet werden. Besonders wirtschaftlich sind Verfahren zur Herstellung der Oligomere, wenn Gemische von α-Olefin und Olefinen mit innenständiger Doppelbindung und ggf. Paraffinen eingesetzt werden.

Für die Verwendung der durch Oligomerisierung hergestellten Produkte ist häufig deren Verzweigungsgrad ein Kriterium. Ein Maß für den Verzweigungsgrad ist der Isoindex. Er ist definiert durch die Anzahl der Verzweigungen pro Molekül. So haben beispielsweise lineare Octene (n-Octene) einen Isoindex von 0, Methylheptene einen von 1 und Dimethylhexene einen von 2. Bei der Berechnung des Isoindex von Gemischen sind die Massenanteile der einzelnen Verbindungsgruppen zu berücksichtigen. Je niedriger der Isoindex eines Gemisches ist, umso linearer sind im Durchschnitt die darin enthaltenden Verbindungen.

Durch den Isoindex eines Olefingemisches ist der geringstmögliche Verzweigungsgrad der Folgeprodukte vorgegeben und somit deren anwendungstechnisches Eigenschaftsprofil mitbestimmt.

Für die Herstellung von Nonanolen durch Hydroformylierung eines C₈-Olefingemisches und anschließende Hydrierung ist eine hohe Linearität des Gemisches vorteilhaft, da die linearen Olefine schneller und selektiver als die verzweigten Olefine reagieren und damit höhere Ausbeuten ergeben. Der Einsatz von einem C₈-Olefingemisch mit niedrigem Isoindex ergibt ein lineareres Nonanolgemisch als beim Einsatzes eines verzweigteren C₈-Olefingemisches**.** Ein geringer Isoindex von Nonanolgemischen verbessert die anwendungstechnischen Eigenschaften der daraus hergestellten Weichmacher, insbesondere die Viskosität. So wirkt sich zum Beispiel bei einem Nonylphthalatgemisch ein niedriger Isoindex günstig in Bezug auf eine niedrige Flüchtigkeit und eine bessere Kältebruch-Temperatur des mit dem Weichmacher hergestellten Weich-PVC aus.

Die Oligomerisierung von Olefinen, insbesondere von Propen und Butenen, wird großtechnisch entweder in homogener Phase an einem molekularen Katalysator oder heterogen an einem festen Katalysator durchgeführt.

Eine homogenkatalytische Arbeitsweise ist z. B. durch A. Chauvel und G. Lefebvre in Petrochemical Processes, Band 1, Editions Technip 1989, pp. 183 - 187, beschrieben. Ein weltweit ausgeübtes homogenkatalytisches Verfahren ist die Oligomerisation mit löslichen molekularen Nickel-Komplexen, bekannt als DIMERSOL-Verfahren (s. Yves Chauvin, Helene Olivier, in "Applied Homogeneous Catalysis with Organometallic Compounds", edited by Boy Comils, Wolfgang A. Herrmann, Verlag Chemie, 1996, pp. 258-268).

Der Nachteil homogenkatalytischer Verfahren besteht darin, dass der Katalysator den Reaktor mit den Reaktionsprodukten und nicht umgesetzten Edukten verlässt und von diesen abgetrennt werden muss. Dies erfordert Aufarbeitungsschritte und verursacht Abfallströme. Eventuell entstehende Abbauprodukte des Katalysators können nicht in situ zum aktiven Katalysator regeneriert werden, wodurch zusätzliche Katalysatorkosten anfallen.

Diese Nachteile bestehen bei heterogenkatalysierten Olefinoligomerisierungsverfahren nicht. Die Oligomerisierung an sauren Kontakten, technisch werden z. B. Zeolithe oder Phosphorsäure auf Trägern eingesetzt, ist lange bekannt. Hierbei werden Isomerengemische von verzweigten Oligomeren erhalten. Selbst unter optimierten Bedingungen bleiben bei der Oligomerisierung von linearen Butenen die stark verzweigten Dimethylhexene das Hauptprodukt. Ein Beispiel für die saure Katalyse von Oligomerisierungen von Olefinen findet sich in WO 92/13818.

Für die nicht-saure, heterogen katalysierte Oligomerisierung von Olefinen werden in der Technik häufig Nickelverbindungen auf Trägermaterialien eingesetzt. Ein Katalysator dieser Art ist ein Nickel-Festbett-Katalysator, der im OCTOL-Prozess des Anmelders eingesetzt wird (Hydrocarbon Process., Int. Ed. 1986, Vol. 65, pp. 31 -33).

Weitere Nickel-Festbett-Katalysatoren mit diesen Eigenschaften sind beispielsweise in DE 43 39 713, in WO 95/14647 und in WO 99/25668 beschrieben.

Die nicht-saure, heterogen katalysierte Oligomerisierung von Olefinen führt unter optimierten Bedingungen zu Produkten mit einer höheren Linearität als mit den sauren heterogenen Kontakten. Nichtsdestotrotz ist die die Linearität der Produkte eingeschränkt. So ist beispielsweise der Isoindex bei dem eingesetzten Octol-Prozess des Anmelders größer 1 und es entstehen ca. 15 % bis 30 % der unerwünschten Dimethylhexene. Homogene molekulare Katalysatoren besitzen durch Variationsmöglichkeiten der Liganden und ihrer stärker definierten Struktur ein größeres Potential für Optimierungen und führen dadurch häufig zu Oligomerisierungskatalysatoren, deren Selektivität zu linearen Produkten höher ist und bei denen weniger Dimethylhexene als Reaktionsprodukte entstehen.

Ein Nachteil, der sowohl bei homogenen als auch bei heterogenen Katalysatoren auftritt ist die stark sinkende Selektivität zu dimeren Reaktionsprodukten bei hohen Umsätzen. Insbesondere bei steigenden Umsätzen steigt der Anteil an trimeren, tetrameren und höheren Oligomeren stark an. Dies führt zu einem erhöhten Aufwand bei der Produktabtrennung und zu Ausbeuteverlust oder, falls die höheren Oligomere anderweitig verwendet werden können, was nur bei großen Oligomerisierungsanlagen wirtschaftlich ist, zu einem höheren Aufwand bei Logistik und Lagerung. Insbesondere bei kleineren Eduktströmen ist es demzufolge Vorteilhaft, wenn keine oder nur in geringem Maße Trimere, Tetramere und höhere Oligomere bei der Reaktion entstehen.

Eine besonders hohe Selektivität zu dimeren Oligomeriserungsprodukten wird häufig durch die Anwendung einer 2-phasigen Reaktionsführung erreicht. Der Katalysator befindet sich hier in einer polaren Phase, während die Produkte sich in einer apolaren Phase befinden oder diese selber bilden. Die als Reaktionsprodukte gewünschten Dimere stehen in der Katalysatorphase also nur in geringem Maße für Konsekutivreaktionen zu höheren Oligomeren zur Verfügung. Ein Beispiel für die Anwendung einer 2-phasigen Reaktionsführung mit einer Ionischen Flüssigkeit als polare Katalysatorphase ist das sog. DIFASOL-Verfahren (Gilbert et al., Oil & Gas Science and Technology - Rev. IFP 2007, Vol. 62, pp. 745 - 759).

Nachteile der 2-phasigen Reaktionsführung sind jedoch die limitierte Konzentration der Eduktolefine in der Katalysatorphase (insbesondere bei Edukten mit mehr als 4 Kohlenstoffatomen), der langsame Stoffaustausch, die Notwendigkeit der Phasentrennung zwischen Reaktions- und Aufarbeitungsteil des Prozesses und häufig die Notwendigkeit großer Mengen Lösemittel.

Es bestand daher die Aufgabe, die Vorteile einer heterogen katalysierten Reaktion, wie z. B. eine unaufwendige Weiterverarbeitung des Reaktionsgemisches und eine einfache Produktabtrennung, mit den Vorteilen einer homogen katalysierten Reaktion, wie z. B. hohe Umsätze und eine einfachere zu höheren Selektivitäten führende Optimierbarkeit, zu kombinieren.

Es wurde nun gefunden, dass Supported lonic Liquid Phase-Katalysatoren - kurz: SILP-Katalysatoren - in der Oligomerisierung von C₃- bis C₅-Olefinen genutzt werden können.
SILP-Katalysatoren sind stückige Katalysatoren, die aus einem festen Trägermaterial bestehen, die mit einer ionischen Flüssigkeit umhüllt ist, in der die aktive katalytische Zusammensetzung gelöst ist. Das Prinzip der Immobilisierung eines molekularen Katalysators in einem Film einer Ionischen Flüssigkeit auf einem festen Träger wurde als erstes in WO 2002098560 beschrieben.

Gegenstand der vorliegenden Erfindung ist ein Katalysatorsystem, bestehend aus:
a) einem Trägermaterial, ausgewählt aus mindestens einer der folgenden Materialien:
   Siliziumdioxid, Aluminiumoxid, Magnesiumoxid, Zirkonoxid sowie deren Mischoxiden, Kohlenstoff-Nanoröhren;
b) einer Ionischen Flüssigkeit;
c) einer katalytisch wirksamen Zusammensetzung, enthaltend Nickel;
d) einem Aktivator, ausgewählt aus der Gruppe der Lewis-Säuren mit alkylierenden Eigenschaften.

Ein weiterer Gegenstand der vorliegenden Erfindung ist ein Verfahren zur Oligomerisierung von Olefinen mit 3 bis 5 Kohlenstoffatomen unter Verwendung der erfindungsgemäßen Katalysatoren.

Insbesondere ist Gegenstand der Erfindung ein Verfahren zur Herstellung eines Gemisches von C₈-Olefinen aus einer Mischung von 1-Buten, cis- und trans-2-Buten sowie der unreaktiven Komponente n-Butan.

Die vorliegende Erfindung weist gegenüber herkömmlichen Verfahren folgende Vorteile auf:
a) Als stückige Katalysatoren haben SILP-Katalysatoren die typischen Vorteile von heterogenen Katalysatoren wie in Jens Hagen, Industrial Catalysis: A Practical Approach, 2006, 2. Ed., Wiley-VCH, p. 12 beschrieben.
b) Im Gegensatz zu 2-Phasen-Systemen sind keine Phasentrennung zwischen Reaktions- und Aufarbeitungsteil sowie Lösemittel notwendig.
c) Da der eigentliche Katalysator ein molekularer Komplex ist, haben die SILP-Katalysatoren die typischen Vorteile von homogenen Katalysatoren wie in Jens Hagen, Industrial Catalysis: A Practical Approach, 2006, 2. Ed., Wiley-VCH, p. 12 beschrieben.
d) Ein besonderer Vorteil beim erfindungsgemäßen Verfahren ist darüber hinaus die hohe Linearität der Produkte und insbesondere die geringe Bildung von Dimethylhexenen.
e) Da die katalytisch aktive Komponente in einer polaren Phase vorliegt, ist die Selektivität zu dimeren Reaktionsprodukten sehr hoch. Die polare Phase ist jedoch nur ein dünner Film, so dass der Stofftransport zumeist nicht limitierend wirkt.

### Nachfolgend wird das erfindungsgemäße Verfahren näher beschrieben.

### a) Trägermaterial

Als Trägermaterialien für die erfindungsgemäßen Katalysatoren eignen sich Kohlenstoff-Nanoröhren, Aktivkohle, Magnesiumoxid, Aluminiumoxid, Zirkonoxid und Siliziumdioxid sowie deren Mischoxide. Die Trägermaterialien können zur Einstellung der Acidität bis zu 1,5 Massen-% Alkalimetalloxide enthalten. Darüber hinaus können Hydroxyl-Gruppen an der Oberfläche der Trägermaterialien mit Organosilizium-Resten geschützt bzw. dehydoxyliert sein.

Bevorzugt werden Siliziumdioxid und Aktivkohle als Trägermaterial verwendet. Besonders bevorzugt werden Siliziumdioxid mit einer Partikelgröße zwischen 0,05 mm und 4 mm und einer BET-Oberfläche von 250 bis 1000 m²/g, bestimmt nach DIN 66131 und 66132 sowie Aktivkohle mit einer BET-Oberfläche zwischen 1000 und 4000 m²/g verwendet. Ganz besonders bevorzugt wird Kieselgel mit einer Partikelgröße zwischen 0,063 mm und 0,2 mm und einer BET-Oberfläche zwischen 300 und 800 m²/g verwendet.

### b) Ionische Flüssigkeit

Als Ionische Flüssigkeit für die erfindungsgemäßen Katalysatoren werden Verbindungen verwendet, wobei das Anion ausgewählt ist aus der Gruppe Tetrafluoroborat ([BF₄]⁻), Hexafluorophosphat ([PF₆]⁻), Dicyanamid ([N(CN)₂]⁻), Bistrifluoromethylsulfonylamid ([NTf₂]⁻), Tricyanomethid ([C(CN)₃]⁻), Tetracyanoborat ([B(CN)₄]⁻), Halogenide (Cl⁻, Br⁻, F⁻, l⁻), Hexafluoroantimonat ([SbF₆]⁻), Hexafluoroarsenat ([AsF₆]⁻), Sulfat ([SO₄]²-), Tosylat ([C₇H₇SO₃]⁻), Nonaflat ([C₄F₉SO₃⁻), Tris-(Pentafluoroethyl)-trifluorophosphat ([PF₃(C₂F₅)₃]⁻), Thiocyanat ([SCN]⁻), Carbonat ([CO₃]²⁻), [R'-COO]⁻, [R'-SO₃]⁻, [R'PO₄R"]⁻ oder [(R'-SO₂)2N]⁻, und R' und R" gleich oder ungleich, jeweils ein linearer oder verzweigter 1 bis 12 Kohlenstoffatome enthaltender aliphatischer oder alicyclischer Alkyl- oder ein C₅-C₁₈- substituierter Aryl-, C₅-C₁₈- substituierter Aryl-C₁-C₆-alkyl- oder C₁-C₆-Alkyl-C₅-C₁₈- substituierter Aryl-Rest ist, der durch Halogenatome substituiert sein kann. Weiterhin kann das Anion eingebracht werden durch Mischen von Halogeniden (Cl⁻, Br⁻, F⁻, I⁻) mit Lewis-sauren Verbindungen, wie beispielsweise Aluminiumverbindungen der allgemeinen Summenformel Al₂XₙR₆₋ₙ mit n = 0-6, mit X = Cl⁻ oder Br⁻ und R = C₁-C₆-alkyl oder C₅-C₁₂-cycloalkyl oder Mischungen dieser Verbindungen, wobei der molare Anteil an Aluminiumhalogeniden größer oder gleich des Anteils der Halogenide sein muss.
Das Kation ist ausgewählt aus:
- quarternären Ammonium-Kationen der allgemeinen Formel [NR¹R²R³R⁴]⁺,
- Phosphonium-Kationen der allgemeinen Formel [PR¹R²R³R⁴]⁺,
- Imidazolium-Kationen der allgemeinen Formel 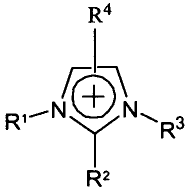 wobei der Imidazol-Kern substituiert sein kann mit wenigstens einer Gruppe, die ausgewählt ist aus C₁-C₆-Alkyl-, C₁-C₆-Alkoxy-, C₁-C₆-substituierte Aminoalkyl-, C₅-C₁₂- substituierte Aryl- oder C₅-C₁₂- substituierte Aryl-C₁-C₆-Alkylgruppen, Pyridinium-Kationen der allgemeinen Formel 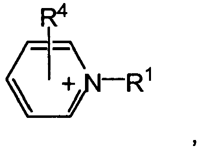 wobei der Pyridin-Kern substituiert sein kann mit wenigstens einer Gruppe, die ausgewählt ist aus C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen; Pyrazolium-Kationen der allgemeinen Formel 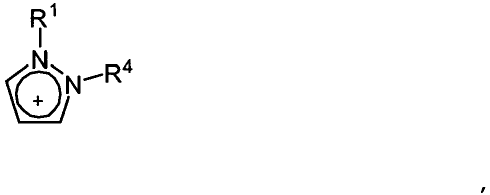 wobei der Pyrazol-Kern substituiert sein kann mit wenigstens einer Gruppe, die ausgewählt ist aus C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen; und Triazolium-Kationen der allgemeinen Formel 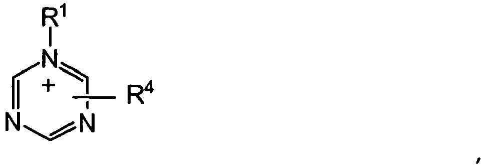 wobei der Triazol-Kern substituiert sein kann mit wenigstens einer Gruppe, die ausgewählt ist aus C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen, und die Reste R¹, R², R³ unabhängig voneinander ausgewählt sind aus der Gruppe bestehend aus:
- Wasserstoff;
- linearen oder verzweigten, gesättigten oder ungesättigten, aliphatischen oder alicyclischen Alkylgruppen mit 1 bis 20 Kohlenstoffatomen;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein können;
- Aryl-, Aryl-C₁-C₆-Alkylgruppen mit 5 bis 12 Kohlenstoffatomen im Arylrest, die gegebenenfalls mit wenigstens einer C₁-C₆-Alkylgruppen und/oder einem Halogenatomen substituiert sein können;
   und der Rest R ausgewählt ist aus:
- linearen oder verzweigten, gesättigten oder ungesättigten, aliphatischen oder alicyclischen Alkylgruppen mit 1 bis 20 Kohlenstoffatomen;
- Heteroaryl-C₁-C₆-Alkylgruppen mit 4 bis 8 Kohlenstoffatomen im Arylrest und wenigstens einem Heteroatom ausgewählt aus N, O und S, die mit wenigstens einer C₁-C₆-Alkylgruppen und/oder Halogenatomen substituiert sein können;
- Aryl-C₁-C₆-Alkylgruppen mit 5 bis 12 Kohlenstoffatomen im Arylrest, die gegebenenfalls mit wenigstens einer C₁-C₆-Alkylgruppe und/oder einem Halogenenatomen substituiert sein können.

Im erfindungsgemäßen Verfahren werden bevorzugt Ionische Flüssigkeiten mit folgenden Anionen verwendet:
Mischungen von Halogeniden (Cl⁻, Br⁻, F⁻, I⁻) mit Aluminiumhalogeniden (AlX₃ mit X=Cl, Br, F, I), wobei der molare Anteil an Aluminiumhalogeniden größer oder gleich des Anteils der Halogenide sein muss, Tetrafluoroborat ([BF₄]⁻), Hexafluorophosphat ([PF₆]⁻), Bistrifluoromethylsulfonylamid ([NTf₂]⁻), Hexafluoroantimonat ([SbF₆]⁻), Hexafluoroarsenat ([AsF_{6]}⁻), Tris-(Pentafluoroethyl)-trifluorophosphat ([PF₃(C₂F₅)₃]⁻).
Besonders bevorzugt werden Mischungen von Halogeniden (Cl⁻, Br⁻, F⁻, I⁻) mit Lewis-sauren Verbindungen eingesetzt, wie beispielsweise Aluminiumverbindungen der allgemeinen Summenformel Al₂XₙR₆₋ₙ mit n = 0-6, mit X = Cl⁻ oder Br⁻ und R = C₁-C₆-alkyl oder C₅-C₁₂-cycloalkyl oder Mischungen dieser Verbindungen, wobei der molare Anteil an Aluminiumhalogeniden größer oder gleich des Anteils der Halogenide sein muss.
Ganz besonders bevorzugt werden Mischungen von Chloridanionen mit Aluminiumtrichlorid mit einem molaren Anteil des Aluminiumtrichlorids von 55% oder das Anion Tris-(Pentafluoroethyl)-trifluorophosphat ([PF₃(C₂F₅)₃]⁻) (FAP) eingesetzt.

Im erfindungsgemäßen Verfahren werden Ionische Flüssigkeiten mit den nachfolgenden Kationen bevorzugt verwendet: 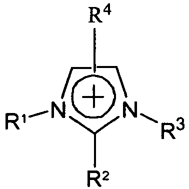 wobei der Imidazol-Kern substituiert sein kann mit wenigstens einer Gruppe, die ausgewählt ist aus C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen. Besonders bevorzug eingesetzt wird das C₁-C₄-Alkyl substituierte Imidazol-Kation.

### c) Katalytisch aktive Zusammensetzung

In der Ionischen Flüssigkeit des stückigen Katalysators ist die katalytisch aktive Zusammensetzung, bestehend aus einem Nickel-Komplex, dessen Vorläufer(n) und gegebenenfalls einem Aktivator, gelöst. Die katalytisch aktive Zusammensetzung wird im erfindungsgemäßen Verfahren aus einem oder mehreren Vorläufern und ggf. einem Aktivator unter Prozessbedingungen erzeugt oder bereits als aktiver Katalysator dem Prozess zugeführt.

### d)Vorläufer

Als Vorläufer zum katalytisch aktiven Nickel-Komplex können alle in der Ionischen Flüssigkeit löslichen Nickelverbindungen mit den formalen Oxidationsstufen 0 oder +2 verwendet werden. Bevorzugt eingesetzt werden NiX₂ (mit X = Cl, Br), Ni(acetylacetonato)₂, Ni(hexafluoroacetylacetonato)₂, Ni(cyclooctadienyl)₂, Ni(cyclopentadienyl)₂, Ni₂(η³-allyl)(µ-Cl₂), Ni₂(η³-allyl)(µ-Br₂), Ni₂(η³-methallyl)(µ-Cl₂), Ni₂(η³-methallyl)(µ-Br₂), Ni(η³-allyl)₂, Ni(η³-methallyl)₂, NiX₂(PR¹R²R³)₂ (mit X = Cl, Br und R¹, R², R³ = C₁-C₈-Alkyl, C₅-C₁₂-Aryl, wobei R¹, R², R³ nicht gleich sein müssen). Besonders bevorzugt wird NiCl₂(PPh₃)₂ eingesetzt.

Als zusätzlicher Vorläufer können gegebenenfalls Phosphorverbindungen mit der formalen Oxidationsstufe +3 eingesetzt werden. Bevorzugt eingesetzt werden Verbindungen der allgemeinen Formel PR¹R²R³ mit R¹, R², R³ = C₁-C₈-Alkyl, C₅-C₁₂- substituierter Arylrest, Verbindungen der allgemeinen Formel P(XR¹)(XR²)(XR³) mit R¹, R², R³ = C₁-C₈-Alkyl, C₅-C₁₂- substituierter Arylrest und X = N, O. Besonders bevorzugt eingesetzt werden Verbindungen der allgemeinen Formel PR¹R²R³ mit R¹, R², R³ = C₅-C₁₂- substituierter Arylrest.

Als zusätzlicher Vorläufer können ggf. 1,3-Diketone eingesetzt werden. Bevorzugt eingesetzt werden 1,1,1,5,5,5-Hexafluoroacetylaceton, 2,2,2-Trifluor-N-(2,2,2-trifluoroacetyl)-acetamid, Acetylaceton.

### d) Aktivator

Als Aktivator können im erfindungsgemäßen Verfahren Lewis-Säuren mit alkylierenden Eigenschaften eingesetzt werden. Bevorzugt eingesetzt werden Verbindungen mit der allgemeinen Summenformel Al₂XₙR₆₋ₙ mit n = 1-5, R = C₁-C₆-Alkyl oder C₅-C₁₂- substituierter Cycloalkylrest und X = Cl- oder Br⁻. Ganz besonders bevorzugt eingesetzt wird Ethylaluminiumdichlorid.

### Verhältnis der Komponenten zueinander

Generell hängt das realisierbare Massenverhältnis von Ionischer Flüssigkeit zu Trägermaterial von Stoffeigenschaften ab. Für die Ionische Flüssigkeit ist besonders ausschlaggebend die Dichte, für das Trägermaterial das Porenvolumen. Im Speziellen hat die Porenradienverteilung des Trägers, das Benetzungsverhalten der Ionischen Flüssigkeit auf der Trägeroberfläche einen Einfluss.

In Abhängigkeit des Trägermaterials und der Art der Ionischen Flüssigkeit, kann die ionische Flüssigkeit in einem weiten Massenverhältnis zum Träger aufgebracht werden. Für Siliziumdioxidträger, deren Hydroxyl-Gruppen an der Oberfläche der Trägermaterialien mit Organosilizium-Resten geschützt bzw. dehydroxyliert sind, können Massenverhältnisse von der bevorzugt verwendeten Ionischen Flüssigkeit (1-Butyl-3-Methylimidazoliumchlorid/Aluminiumchlorid) zum Träger von 0,007 bis 0,674 verwendet werden. Bevorzugt werden Massenverhältnisse von 0,034 bis 0,334. Besonders bevorzugt wird der Bereich von 0,067 bis 0,169. Für Aktivkohleträger werden Verhältnisse von 0,022 bis 2,171 bevorzugt verwendet. Besonders bevorzugt werden Massenverhältnisse von 0,217 bis 0,543. Für die besonders bevorzugten Siliziumdioxidträger werden Massenverhältnisse von Ionischer Flüssigkeit zu Trägermaterial von 0,012 bis 1,255 bevorzugt verwendet. Besonders bevorzugt werden Verhältnisse von 0,063 bis 0,628. Ganz besonders bevorzugt werden Verhältnisse von 0,126 bis 0,314.

Die realisierbaren Massenverhältnisse von Nickel zu Ionischer Flüssigkeit sind generell von der Art der ionischen Flüssigkeit und von der Art des Trägermaterials abhängig.
Für die besonders bevorzugten Siliziumdioxidträger werden für die besonders bevorzugten Massenverhältnisse von Ionischer Flüssigkeit zu Trägermaterial von 0,126 bis 0,314. Massenverhältnisse von Nickel zu Ionischer Flüssigkeit von 0,003 bis 0,027 verwendet. Bevorzugt werden Verhältnisse von 0,010 bis 0,020. Besonders bevorzugt wird das Verhältnis von 0,010 bis 0,011.

### Katalysatorherstellung

Die erfindungsgemäßen Katalysatoren können durch Behandlung des festen Trägermaterials mit einer homogenen Lösung, die die anderen Katalysatorkomponenten und ggf. ein Lösemittel enthält, erfolgen.

Ein Weg ist das Tränken des festen Trägermaterials mit einer solchen Lösung. Dabei wird das Trägermaterial mit einem Überschuss an Lösung versetzt. Nach Stoffaustausch zwischen Trägermaterial und Lösung, der durch Schütteln oder Rühren beschleunigt werden kann, wird die überschüssige Flüssigkeit mechanisch vom stückigen Katalysator abgetrennt, beispielsweise durch Filtration oder Zentrifugieren. Gegebenenfalls folgt ein Trocknungsschritt.

Eine andere Methode ist die Besprühung des Trägermaterials mit einer solchen Lösung. Gegebenenfalls wird ein zugesetztes Lösemittel während des oder nach dem Sprühvorgang(s) entfernt.

Bevorzugt wird der Katalysator durch Versetzen des Trägermaterials mit einer Lösung, die aus den anderen Katalysatorkomponenten und einem Lösemittel besteht, und Verdampfen des Lösemittels hergestellt. Als Lösemittel dafür werden bevorzugt leichtsiedende Stoffe eingesetzt, die mit den anderen Katalysatorkomponenten eine homogene Lösung bilden und die mit keiner dieser Komponenten reagieren. Geeignete Lösemittel sind beispielsweise Dichlormethan, Hexan und Toluol. Ein ganz besonderes bevorzugtes Lösemittel ist Dichlormethan.

Die Abtrennung des Lösemittels erfolgt im Druckbereich von 10⁵ bis 2*10² Pa, wobei ausgehend von Normaldruck sukzessiv der Druck gesenkt wird. Um Zersetzung des Katalysators während der Katalysatorherstellung zu vermeiden, erfolgt die Herstellung unter Sauerstoff- und Wasserausschluss.

Die erfindungsgemäßen Trägerkatalysatoren werden vorzugsweise in einer Form hergestellt, die bei der Oligomerisierung einen geringen Strömungswiderstand bietet. Typische Formen sind Tabletten, Zylinder, Strangextrudate oder Ringe. Die Formgebung erfolgt dabei im Regelfall am Trägermaterial vor Aufbringen der Ionischen Flüssigkeit und der darin gelösten Katalysatorkomponenten. Es können auch granulatförmige Träger zur Herstellung der erfindungsgemäßen Katalysatoren eingesetzt werden. Durch Aussieben kann dabei ein Katalysatorträger mit der gewünschten Korngröße abgetrennt werden. Häufig können Trägermaterialien als Formkörper käuflich erworben werden.

### Einsatzstoffe

An den erfindungsgemäßen Katalysatoren können Olefine mit 3 bis 5 Kohlenstoffatomen zu Olefinen mit 6 bis 10 Kohlenstoffatomen oligomerisiert werden. Es können prinzipiell alle Olefine oder Olefingemische mit 3 bis 5 Kohlenstoffatome unabhängig von der Lage der Doppelbindung eingesetzt werden. Die Einsatzstoffe können aus Olefinen gleicher, ähnlicher (±2) oder deutlich unterschiedlicher (> 2) C-Zahl bestehen.

Es sei darauf hingewiesen, dass nicht nur die oben genannten Olefine, sondern auch deren Mischungen mit gesättigten Kohlenwasserstoffen eingesetzt werden können.

Im erfindungsgemäßen Verfahren werden bevorzugt 2-Butene und 1-Buten eingesetzt. Insbesondere eingesetzt werden Mischungen aus 2-Butenen, 1-Buten und linearen sowie verzweigten Butanen, wie sie bei der Aufarbeitung des C₄-Schnitts eines Steamcrackers nach Abtrennung des der mehrfach ungesättigten Verbindungen und des Isobutens entsteht. Diese Techniken sind in der Fachliteratur beschrieben (K. Weissermel, H. J. Arpe, Industrielle Organische Chemie, Wiley-VCH, 5. Auflage, 1998).

### Verfahrensdurchführung

### Beispiele

Die folgenden Beispiele sollen die Erfindung erläutern.

### Beispiel 1, erfindungsgemäß: Herstellung eines SILP-Katalysators mit der Ionischen Flüssigkeit (IL) 1-Butyl-3-Methylimidazoliumchlorid ([BMIM]Cl) / Aluminiumchlorid und dem Katalysatorvorläufer NiCl₂(PPh₃)₂

Alle Arbeitsschritte werden in der Inertgasbox oder mittels Schlenktechnik durchgeführt. Zur Herstellung einer Katalysatorcharge für einen Batchversuch werden 147,1 mg NiCl₂(PPh₃)₂ (kommerziell erhältlich) und 471,6 mg Triphenylphosphin (kommerziell erhältlich) in einen 100 ml Schlenkkolben eingewogen. Es werden 20 ml trockenes Dichlormethan (Wassergehalt < 20 ppm) zugegeben und für 15 Minuten auf einem Magnetrührer gerührt. Anschließend werden 4,0 g Kieselgel (Partikelgröße 0,063 - 0,200 mm, BET-Oberfläche 315 m²/g, Porenvolumen 0,996 ml/g) hinzugefügt und die Suspension weitere 15 Minuten gerührt. Dann werden 1,26 g [BMIM]Cl/AlCl₃ (molarer Anteil AlCl₃: 55 %) eingewogen. Nachweiteren 15 Minuten auf dem Magnetrührer wird der Kolben in einem Wasserbad bei Raumtemperatur an eine Vakuumpumpe angeschlossen und der Druck langsam von 10⁵ Pa auf 2*10² Pa reduziert. Nach etwa 45 Minuten liegt ein türkises Pulver vor.

### Beschreibung der Oligomerisierungsapparatur

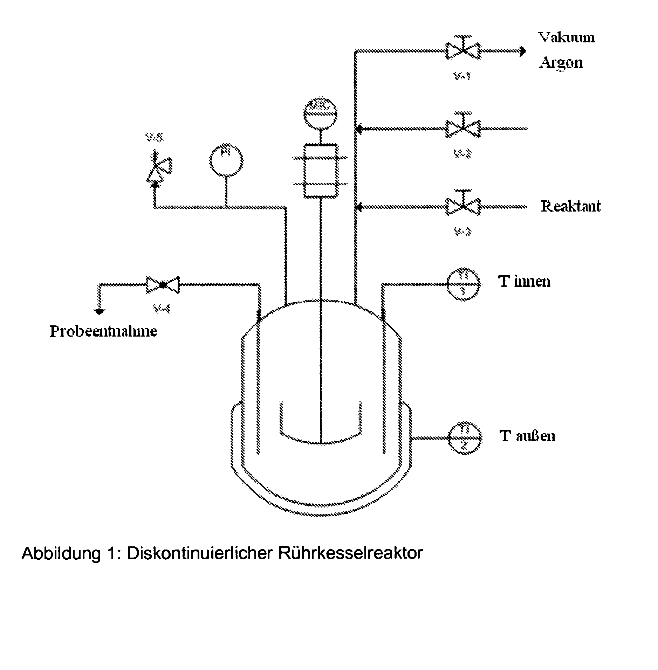

Das Fließbild des zur diskontinuierlichen Dimerisierung verwendeten Rührkesselreaktors, ist in Abbildung 1 dargestellt. Der Hastelloy-Autoklav verfügt über eine motorisch angetriebene Rühreinheit (Gaseintragsrührer, Heidolph RZR 2020 Motor) sowie über eine Druck- (PI) und Temperaturanzeige (TI 1). Das Überdruckventil V-5, das bei 80 bar öffnet, verhindert einen zu hohen, unerwünschten Druckanstieg im Reaktor. Über die Ventile V-1, V-2 und V-3 können Reaktanten sowie Schutzgas in den Rührkessel geleitet werden. Proben können während eines Katalyseversuches über Ventil V-4 entnommen werden.

### Beschreibung der Versuchsdurchführung

Zur Durchführung eines diskontinuierlichen Dimerisierungsversuches wurde wie folgt vorgegangen: Mittels Schlenktechnik wurde das hergestellte SILP-Pulver unter Inertgasbedingungen in den Reaktortopf gegeben und der Reaktor verschraubt. Durch eine Deckelöffnung wurden 80 ml Cyclohexan (Wassergehalt < 20 ppm) eingefüllt. Etwa 1 ml n-Nonan als interner GC-Standard und 6 ml Ethylaluminiumdichlorid (0,1 molare Lösung in Hexan, kommerziell erhältlich) wurden zugegeben und die Deckelöffnung verschlossen.

An V-3 wurde ein Stahlzylinder mit 22 g Edukt angeschlossen und kurz vor Versuchsbeginn in den Reaktor eingeleitet. Mit Starten des Rührers mit 1000 1/min begann die Zeitnahme. Über Ventil V-4 wurden Proben aus dem Reaktor entnommen. Die Proben wurden vor der gaschromatographischen Analyse hydriert. So konnten leicht die unterschiedlich verzweigen Produkte voneinander getrennt werden.

### Erfindungsgemäße Versuchsergebnisse

Die Herstellung der katalytisch aktiven Zusammensetzung und die Versuchsdurchführung entsprachen den oben beschriebenen Prozeduren. Als Edukt kam ein Gemisch aus 70 % n-Butan, 19 % trans-2-Buten, 9 % cis-2-Buten, 1.7 % 1-Buten und 0.3 % Isobutan+C5-Alkane zum Einsatz. Die Eduktmasse bezieht sich auf das Gemisch. Der Umsatz ist nur auf die enthaltenen Butene bezogen.

**Tabelle 1: Beispielhafte Versuchsergebnisse mit dem erfindungsgemäßen Katalysatorsystem: Katalysatorvorläufer NiCl₂(PPh₃)₂; Temperatur: 20 °C; Aktivator: 6 ml EtAlCl₂-Lösung (0,1 molare Lösung in Hexan)**

| Nr. | Ionische Flüssigkeit | Trägermaterial | IL/Trägermaterial | Umsatz 60 min | S_{C8} | Iso-Index | S_{DM H} | S_{MH} | S_{Oct} |
|---|---|---|---|---|---|---|---|---|---|
| | | | [-] | [%] | [%] | [-] | [%] | [%] | [%] |
| 1 | [BMIM]Cl/AlCl₃ | Silica | 0,126 | 69,4 | 94,2 | 1,0 | 11,6 | 76,8 | 11,6 |
| 2 | [EMIM][FAP] | Silica | 0,171 | 12,6 | 98,8 | 0,97 | 9,9 | 77,1 | 13,0 |
| 3 | [BMIM]Cl/AlCl₃ | A-Kohle | 0,217 | 40,3 | 94,2 | 0,96 | 7,6 | 81,0 | 11,4 |
| 4 | [BMIM]Cl/AlCl₃ | Si-Silica | 0,067 | 33,7 | 97,8 | 0,97 | 9,8 | 77,2 | 13,0 |

[BMIM]Cl/AlCl₃= 1-Butyl-3-methylimidazoliumchlorid/Aluminiumchlorid (Verhältnis 1/1.1), [EMIM][FAP]= 1-Ethyl-3-methylimidazolium-tris-(pentafluoroethyl)-trifluorophosphat, Kieselgel: Merck KGaA Typ 10184 (Partikelgröße 0,063 - 0,200 mm, BET-Oberfläche 315 m²/g, Porenvolumen 0,996 ml/g), A-Kohle: Aktivkohle Fluka (Pulver, BET-Oberfläche 2056 m²/g, Porenvolumen 1,723 ml/g), Si-Silica: Silica 60 silanisiert: Merck KGaA (Partikelgröße 0,063 - 0,200 mm, BET-Oberfläche 430 m²/g, Porenvolumen 0,535 ml/g).

Eintrag 1 aus Tabelle 1 zeigt, dass mit den erfindungsgemäßen Katalysatorsystem unter Verwendung von NiCl₂(PPh₃)₂, Ethylaluminiumdichlorid als Aktivator, einer Mischung aus 1-Butyl-3-methylimidazoliumchlorid und Aluminiumchlorid im Verhältnis 1/1.1 als Ionische Flüssigkeit und Kieselgel als Trägermaterial ein C4-Kohlenwasserstoff mit einem niedrigen Olefingehalt von etwa 30 % eine sehr hohe Selektivität von 94 % zu den gewünschten dimeren Reaktionsprodukten bei gleichzeitig niedriger Selektivität (11,6 %) zum unerwünschten Dimethylhexen erzielt werden kann. Der Isoindex beträgt 1.

Eintrag 2 aus Tabelle 1 zeigt die Verwendung von 1-Ethyl-3-methylimidazoliumtris-(pentafluoroethyl)-trifluorophosphat als Ionische Flüssigkeit unter ansonsten gleichen Bedingungen. Hier werden noch höhere Selektivitäten zu den C8-Oligomeren erzielt, wobei der Umsatz jedoch niedriger ist. Die Selektivität zu Dimethylhexenen ist mit etwa 10 % sehr niedrig. Gleichzeitig wird ein sehr guter Isoindex von <1 erzielt.

Eintrag 3 aus Tabelle 1 zeigt die Verwendung von Aktivkohle als Trägermaterial. Hier werden mit 7 % eine besonders niedrige Selektivität zu Dimethylhexenen und ein besonders niedriger Isoindex bei einem Umsatz von 40 % erzielt.

Eintrag 4 aus Tabelle 1 zeigt die Verwendung von durch Silylierung dehydroxyliertem Kieselgel. Hier wird ebenfalls ein sehr guter Isoindex von < 1 erzielt. Das besondere bei diesem Beispiel ist, dass sehr wenig Ionische Flüssigkeit benötigt wird, wie das mit Abstand niedrigste Verhältnis IL/Trägermaterial mit einem Wert von 0,067 verdeutlicht.

### Vergleich mit dem Stand der Technik:

### Vergleich mit klassisch Homogener Katalyse:

Mit dem eingangs erwähnten rein homogen-katalysierten Dimersol-Prozess (s. Yves Chauvin, Helene Olivier, in "Applied Homogeneous Catalysis with Organometallic Compounds", edited by Boy Cornils, Wolfgang A. Herrmann, Verlag Chemie, 1996, pp. 258 - 268) entstehen bis zu 40 % Dimethylhexene. Der Isoindex beträgt typischerweise 1 bis 1,35 und die Selektivität zu C8-Oligomeren ist bei diesem Prozess < 90 %. Das heißt, die erfindungsgemäßen Katalysatorsysteme sind dem Dimersol-Prozess sowohl bezüglich der Selektivität zu C8-Oligomeren als auch der Linearität der Produkte, insbesondere bezüglich einer niedrigen Dimethylhexenselektivität, überlegen. Darüber hinaus ist die Reaktionsführung mit den erfindungsgemäßen Katalysatorsystemen erheblich einfacher, da sie aufgrund ihres immobilisierten Charakters leicht von den Produkten abgetrennt werden können.

### Vergleich mit klassisch Heterogener Katelyse:

Bei dem rein heterogen-katalysierten Prozess beträgt die Selektivität zu Dimethylhexenen etwa 15 % bis 30 % bei einem Isoindex, der typischerweise > 1 ist. Die Dimer-Selektivität ist < 90 % (s. Albers et al., Oligomerisation of C3-C5 on Solid State Nickel Compounds: Complex Catalysts for a versatile Reaction, DGMK-Tagungsbericht 2004-3). Das heißt, die erfindungsgemäßen Katalysatorsysteme sind diesem Prozess sowohl bezüglich der Selektivität zu C8-Oligomeren als auch der Linearität der Produkte, insbesondere bezüglich einer niedrigen Dimethylhexenselektivität, überlegen.

### Flüssig-Flüssig-2-Phasen-Reaktionsweise:

Mit NiCl₂(PPh₃)₂, Ethylaluminiumdichlorid als Aktivator und 1-Butyl-3-methylimidazoliumchlorid/Aluminiumchlorid (Verhältnis 1/1.1) als polarer Phase in einer Flüssig-Flüssig-2-Phasen-Reaktionsweise werden Selektivitäten von 20 % bis 30 % zu Dimethylhexenen und ein Isoindex von > 1 beobachtet (s. Gilbert et al., Oil & Gas Science and Technology - Rev. IFP 2007, Vol. 62, pp. 745 - 759). Die Selektivität zu Dimeren ist mit etwa 95 % ähnlich hoch wie bei den erfindungsgemäßen Katalysatorsystemen. Bezüglich der Linearität der Produkte und der Dimethylselektivität sind die erfindungsgemäßen Katalysatorsysteme jedoch überlegen.

## Patentansprüche

1. Katalysatorsystem, bestehend aus:
a) einem Trägermaterial, ausgewählt aus mindestens einem der folgenden Materialien: Siliziumdioxid, Aluminiumoxid, Magnesiumoxid, Zirkonoxid sowie deren Mischoxiden, Kohlenstoff-Nanoröhren;
b) einer Ionischen Flüssigkeit;
c) einer katalytisch wirksamen Zusammensetzung, enthaltend Nickel;
d) einem Aktivator, ausgewählt aus der Gruppe der Lewis-Säuren mit alkylierenden Eigenschaften.

2. Katalysatorsystem nach Anspruch 1, **dadurch gekennzeichnet, dass** als Ionische Flüssigkeit Verbindungen verwendet werden, wobei das Anion ausgewählt ist aus der Gruppe:
Tetrafluoroborat ([BF₄]⁻), Hexafluorophosphat ([PF₆]⁻), Dicyanamid ([N(CN)₂]⁻), Bistrifluoromethylsulfonylamid ([NTf₂]⁻), Tricyanomethid ([C(CN)₃]⁻), Tetracyanoborat ([B(CN)₄]⁻), Halogenide (Cl⁻, Br⁻, F⁻, I⁻), Hexafluoroantimonat ([SbF₆]⁻), Hexafluoroarsenat ([AsF₆]⁻), Sulfat ([SO₄]²⁻), Tosylat ([C₇H₇SO₃]⁻), Nonaflat ([C₄F₉SO₃⁻), Tris-(Pentafluoroethyl)-trifluorophosphat ([PF₃(C₂F₅)₃]⁻), Thiocyanat ([SCN]⁻), Carbonat ([CO₃]²⁻), [R'-COO]⁻, [R'-SO₃]⁻, [R'PO₄R"]⁻ oder [(R'-SO₂)2N]⁻, und R' und R" gleich oder ungleich, jeweils ein linearer oder verzweigter 1 bis 12 Kohlenstoffatome enthaltender aliphatischer oder alicyclischer Alkyl- oder ein C₅-C₁₈- substituierter Aryl-, C₅-C₁₈- substituierter Aryl-C₁-C₆-alkyl- oder C₁-C₆-Alkyl-C₅-C₁₈- substituierter Aryl-Rest ist, der durch Halogenatome substituiert sein kann;
wobei das Kation ausgewählt ist aus:
quarternären Ammonium-Kationen der allgemeinen Formel [NR1 R2R3R4]+; Phosphonium-Kationen der allgemeinen Formel [PR1 R2R3R4]+ oder:
Imidazolium-Kationen der allgemeinen Formel 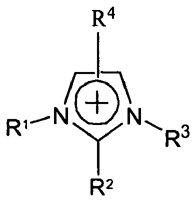 wobei der Imidazol-Kern substituiert sein kann mit wenigstens einer Gruppe, die ausgewählt ist aus C1-C6-Alkyl-, C1-C6-Alkoxy-, C1-C6- substituierte Aminoalkyl-, C5-C12- substituierte Aryl- oder C5-C12- substituierte Aryl-C1-C6-Alkylgruppen,
Pyridinium-Kationen der allgemeinen Formel 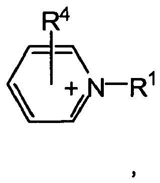 wobei der Pyridin-Kern substituiert sein kann mit wenigstens einer Gruppe, die ausgewählt ist aus C1-C6-Alkyl-, C1-C6-Alkoxy-, C1-C6- substituierte Aminoalkyl-, C5-C12- substituierte Aryl- oder C5-C12- substituierte Aryl-C1-C6-Alkylgruppen,
Pyrazolium-Kationen der allgemeinen Formel 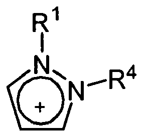 wobei der Pyrazol-Kern substituiert sein kann mit wenigstens einer Gruppe, die ausgewählt ist aus C1-C6-Alkyl-, C1-C6-Alkoxy-, C1-C6- substituierte Aminoalkyl-, C5-C12- substituierte Aryl- oder C5-C12- substituierte Aryl-C1-C6-Alkylgruppen,
und Triazolium-Kationen der allgemeinen Formel 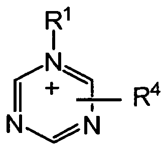 wobei der Triazol-Kern substituiert sein kann mit wenigstens einer Gruppe, die ausgewählt ist aus C1-C6-Alkyl-, C1-C6-Alkoxy-, C1-C6- substituierte Aminoalkyl-, C5-C12- substituierte Aryl- oder C5-C12- substituierte Aryl-C1-C6-Alkylgruppen,
und die Reste R1, R2, R3 unabhängig voneinander ausgewählt sind aus der Gruppe bestehend aus:
Wasserstoff;
linearen oder verzweigten, gesättigten oder ungesättigten, aliphatischen oder alicyclischen Alkylgruppen mit 1 bis 20 Kohlenstoffatomen;
Heteroaryl-, Heteroaryl-C1-C6-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C1-C6-Alkylgruppen und/oder Halogenatomen substituiert sein können;
Aryl-, Aryl-C1-C6-Alkylgruppen mit 5 bis 12 Kohlenstoffatomen im Arylrest, die gegebenenfalls mit wenigstens einer C1-C6-Alkylgruppen und/oder einem Halogenatomen substituiert sein können;
und der Rest R ausgewählt ist aus linearen oder verzweigten, gesättigten oder ungesättigten, aliphatischen oder alicyclischen Alkylgruppen mit 1 bis 20 Kohlenstoffatomen;
Heteroaryl-C1-C6-Alkylgruppen mit 4 bis 8 Kohlenstoffatomen im Arylrest und wenigstens einem Heteroatom ausgewählt aus N, O und S, die mit wenigstens einer C1-C6-Alkylgruppen und/oder Halogenatomen substituiert sein können;
Aryl-C1-C6-Alkylgruppen mit 5 bis 12 Kohlenstoffatomen im Arylrest, die gegebenenfalls mit wenigstens einer C1-C6-Alkylgruppe und/oder einem Halogenenatomen substituiert sein können.

3. Katalysatorsystem nach den Ansprüchen 1 bis 2, **dadurch gekennzeichnet, dass** die katalytisch wirksame Zusammensetzung Nickel in für die verwendete Ionische Flüssigkeit löslicher Form in den Oxidationstufen 0 bis +2 enthält.

4. Katalysatorsystem nach Anspruch 3, **dadurch gekennzeichnet, dass** NiCl₂(P[C₆H₅]₃) verwendet wird.

5. Katalysatorsystem nach den Ansprüchen 1 und 2, **dadurch gekennzeichnet, dass** als Lewis-Säure mit alkylierenden Eigenschaften organische Aluminiumverbindungen der allgemeinen Summenformel Al₂XₙR₆₋ₙ mit n = 0-6, mit X = Cl⁻ oder Br⁻ und R = C₁-C₆-Alkyl oder C₅-C₁₂-Cycloalkyl oder Mischungen dieser Verbindungen verwendet werden.

6. Katalysatorsystem nach den Ansprüchen 1 und 2, **dadurch gekennzeichnet, dass** als Lewis-Säure mit alkylierenden Eigenschaften Ethylaluminiumdichlorid verwendet wird.

7. Katalysatorsystem nach mindestens einem der Ansprüche 1 bis 6, **dadurch gekennzeichnet, dass** eine Zusammensetzung enthaltend 1-Butyl-3-Methylimidazoliumchlorid und Aluminiumchlorid verwendet wird.

8. Katalysatorsystem nach mindestens einem der Ansprüche 1 bis 6, **dadurch gekennzeichnet, dass** 1-Ethyl-3-methylimidazolium-tris-(pentafluoroethyl)-trifluorophosphat verwendet wird.

9. Katalysatorsystem nach mindestens einem der Ansprüche 1 bis 8, **dadurch gekennzeichnet, dass** als Trägermaterial Siliziumdioxid mit einer Partikelgröße von 0,063 bis 0,2 mm, einer BET-Oberfläche von 250 bis 1000 m²/g, bestimmt nach DIN 66131 und 66132, verwendet wird.

10. Katalysatorsystem nach mindestens einem der Ansprüche 1 bis 9, **dadurch gekennzeichnet, dass** das Massenverhältnis von Trägermaterial, enthaltend Siliziumdioxid, zu Ionischer Flüssigkeit nach Anspruch 2 in einem Bereich von 0,012 bis 1,255 liegt.

11. Katalysatorsystem nach mindestens einem der Ansprüche 1 bis 9, **dadurch gekennzeichnet, dass** das Massenverhältnis von Trägermaterial, enthaltend Siliziumdioxid, welches in an sich bekannter Weise zuvor dehydroxyliert wurde, zu Ionischer Flüssigkeit nach Anspruch 2 in einem Bereich von 0,007 bis 0,674 liegt.

12. Katalysatorsystem nach mindestens einem der Ansprüche 1 bis 9, **dadurch gekennzeichnet, dass** das Massenverhältnis von Trägermaterial, enthaltend Aktivkohle mit einer BET-Oberfläche zwischen 1000 m²/g und 4000 m²/g bestimmt nach DIN 66131 und 66132, zu Ionischer Flüssigkeit nach Anspruch 2 in einem Bereich von 0,022 bis 2,171 liegt.

13. Verfahren zur Oligomerisierung von ungesättigten Kohlenwasserstoffgemischen unter Verwendung eines Katalysatorsystems nach mindestens einem der Ansprüche 1 bis 12.

14. Verfahren nach Anspruch 13, **dadurch gekennzeichnet, dass** als ungesättigtes Kohlenwasserstoffgemisch ein Strom enthaltend Olefine mit drei bis fünf Kohlenstoffatomen verwendet wird.

15. Verfahren nach Anspruch 14, **dadurch gekennzeichnet, dass** als ungesättigtes Kohlenwasserstoffgemisch ein Strom enthaltend lineare C4-Olefine und gesättigte Kohlenwasserstoffe verwendet wird.

16. Verfahren nach Anspruch 15, **dadurch gekennzeichnet, dass** als ungesättigtes Kohlenwasserstoffgemisch ein Strom enthaltend gesättigte Kohlenwasserstoffe mit einem Massenanteil zwischen 50 % und 80 % und lineare C4-Olefine verwendet wird.

## Claims

1. A catalyst system composed of:
a) a support material selected from at least one of the following materials: silicon dioxide, aluminium oxide, magnesium oxide, zirconium oxide and mixed oxides thereof, carbon nanotubes;
b) an ionic liquid;
c) a catalytically active composition comprising nickel;
d) an activator selected from the group of Lewis acids with alkylating properties.

2. A catalyst system according to claim 1, **characterized in that** compounds are used as ionic liquid, the anion being selected from the following group:
tetrafluoroborate ([BF₄]⁻), hexafluorophosphate ([PF₆]⁻), dicyanamide ([N(CN)₂]⁻), bistrifluoromethylsulphonylamide ([NTf₂]⁻), tricyanomethide ([C(CN)₃]⁻), tetracyanoborate ([B(CN)4]⁻), halides (Cl⁻, Br⁻, F⁻, I⁻, hexafluoroantimonate ([SbF₆]⁻), hexafluoroarsenate ([AsF₆]⁻), sulphate ([SO4]²⁻), tosylate ([C₇H₇SO₃]⁻), nonaflate ([C₄F₉SO₃⁻), tris(pentafluoroethyl)trifluorophosphate ([PF₃(C₂F₅)₃]⁻), thiocyanate ([SCN]⁻), carbonate ([CO₃]²⁻), [R'-COO]⁻, [R'-SO₃]⁻, [R'PO₄R"]⁻ or [(R'-SO₂)2N]⁻, and R' and R " are identical or non-identical, each being a linear or branched, 1- to 12 carbon-atom-containing, aliphatic or alicyclic alkyl radical or a C₅-C₁₈ substituted aryl, C₅-C₁₈ substituted aryl-C₁-C₆-alkyl or C₁-C₆-alkyl-C₅-C₁₈ substituted aryl radical, which may be substituted by halogen atoms;
the cation being selected from the following:
quaternary ammonium cations of the general formula [NR¹R²R³R4]⁺;
phosphonium cations of the general formula [PR¹R²R³R4]⁺ or:
imidazolium cations of the general formula 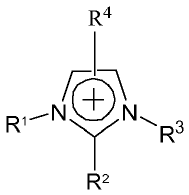 where the imidazole nucleus may be substituted by at least one group selected from C₁-C₆ alkyl, C₁-C₆ alkoxy, C₁-C₆ substituted aminoalkyl, C₅-C₁₂ substituted aryl or C₅-C₁₂ substituted aryl-C₁-C₆ alkyl groups,
pyridinium cations of the general formula 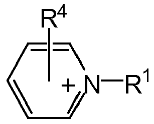 where the pyridine nucleus may be substituted by at least one group selected from C₁-C₆ alkyl, C₁-C₆ alkoxy, C₁-C₆ substituted aminoalkyl, C₅-C₁ substituted aryl or C₅-C₁₂ substituted aryl-C₁-C₆ alkyl groups,
pyrazolium cations of the general formula 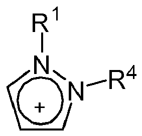 where the pyrazole nucleus may be substituted by at least one group selected from C₁-C₆ alkyl, C₁-C₆ alkoxy, C₁-C₆ substituted aminoalkyl, C₅-C₁₂ substituted aryl or C₅-C₁₂ substituted aryl-C₁-C₆ alkyl groups,
and triazolium cations of the general formula 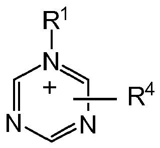 where the triazole nucleus may be substituted by at least one group selected from C₁-C₆ alkyl, C₁-C₆ alkoxy, C₁-C₆ substituted aminoalkyl, C₅-C₁₂ substituted aryl or C₅-C₁₂ substituted aryl-C₁-C₆ alkyl groups,
and the radicals R¹, R², R³ are selected independently of one another from the group consisting of the following:
hydrogen;
linear or branched, saturated or unsaturated, aliphatic or alicyclic alkyl groups having 1 to 20 carbon atoms;
heteroaryl, heteroaryl-C₁-C₆ alkyl groups having 3 to 8 carbon atoms in the heteroaryl radical and at least one heteroatom selected from N, 0 and S, which may be substituted by at least one group selected from C₁-C₆ alkyl groups and/or halogen atoms;
aryl, aryl-C₁-C₆ alkyl groups having 5 to 12 carbon atoms in the aryl radical, which optionally may be substituted by one or more C₁-C₆ alkyl groups and/or halogen atoms;
and the radical R is selected from linear or branched, saturated or unsaturated, aliphatic or alicyclic alkyl groups having 1 to 20 carbon atoms;
heteroaryl-C₁-C₆ alkyl groups having 4 to 8 carbon atoms in the aryl radical and at least one heteroatom selected from N, 0 and S, which may be substituted by one or more C₁-C₆ alkyl groups and/or halogen atoms;
aryl-C₁-C₆ alkyl groups having 5 to 12 carbon atoms in the aryl radical, which optionally may be substituted by one or more C₁-C₆ alkyl groups and/or halogen atoms.

3. A catalyst system according to either of claims 1 and 2, **characterized in that** the catalytically active composition comprises nickel in a form soluble for the ionic liquid used, in the 0 to +2 oxidation states.

4. A catalyst system according to claim 3, **characterized in that** NiCl₂ (P[C₆H₅]₃) is used.

5. A catalyst system according to either of claims 1 and 2, **characterized in that** organic aluminium compounds of the general empirical formula Al₂XₙR₆₋ₙ with n = 0-6, with X = Cl⁻ or Br⁻ and R = C₁-C₆ alkyl or C₅-C₁₂ cycloalkyl or mixtures of these compounds are used as Lewis acid with alkylating properties.

6. A catalyst system according to either of claims 1 and 2, **characterized in that** ethylaluminium dichloride is used as Lewis acid with alkylating properties.

7. A catalyst system according to at least one of claims 1 to 6, **characterized in that** a composition comprising 1-butyl-3-methylimidazolium chloride and aluminium chloride is used.

8. A catalyst system according to at least one of claims 1 to 6, **characterized in that** 1-ethyl-3-methylimidazolium tris(pentafluoroethyl)trifluorophosphate is used.

9. A catalyst system according to at least one of claims 1 to 8, **characterized in that** silicon dioxide having a particle size of 0.063 to 0.2 mm, a BET surface area of 250 to 1000 m²/g, determined in accordance with DIN 66131 and 66132, is used as support material.

10. A catalyst system according to at least one of claims 1 to 9, **characterized in that** the mass ratio of support material comprising silicon dioxide to ionic liquid according to claim 2 is in a range from 0.012 to 1.255.

11. A catalyst system according to at least one of claims 1 to 9, **characterized in that** the mass ratio of support material comprising silicon dioxide which has been dehydroxylated beforehand in a manner known per se to ionic liquid according to claim 2 is in a range from 0.007 to 0.674.

12. A catalyst system according to at least one of claims 1 to 9, **characterized in that** the mass ratio of support material, comprising activated carbon having a BET surface area of between 1000 m²/g and 4000 m²/g as determined in accordance with DIN 66131 and 66132 to ionic liquid according to claim 2 is in a range from 0.022 to 2.171.

13. A process for oligomerizing unsaturated hydrocarbon mixtures using a catalyst system according to at least one of claims 1 to 12.

14. A process according to claim 13, **characterized in that** a stream comprising olefins having three to five carbon atoms is used as unsaturated hydrocarbon mixture.

15. A process according to claim 14, **characterized in that** a stream comprising linear C4 olefins and saturated hydrocarbons is used as unsaturated hydrocarbon mixture.

16. A process according to claim 15, **characterized in that** a stream comprising saturated hydrocarbons with a mass fraction between 50% and 80% and linear C4 olefins is used as unsaturated hydrocarbon mixture.

## Revendications

1. Système catalytique, constitué par :
a) un matériau support, choisi parmi au moins un des matériaux suivantes : dioxyde de silicium, oxyde d'aluminium, oxyde de magnésium, oxyde de zirconium ainsi que leurs oxydes mixtes, nanotubes de carbone ;
b) un liquide ionique ;
c) une composition catalytiquement active, contenant du nickel ;
d) un activateur, choisi dans le groupe des acides de Lewis présentant des propriétés d'alkylation.

2. Système catalytique selon la revendication 1, **caractérisé en ce qu'**on utilise comme liquide ionique des composés, l'anion étant choisi dans le groupe :
tétrafluoroborate ([BF₄]⁻), hexafluorophosphate ([PF₆]⁻), dicyanamide ([N(CN)₂]⁻), bistrifluorométhylsulfonylamide ([NTf₂]⁻), tricyanométhide ([C(CN)₃]⁻), tétracyanoborate ([B(CN)₄]⁻), halogénures (Cl⁻, Br⁻, F⁻, I⁻), hexafluoroantimonate ([SbF₆]⁻), hexafluoroarsénate ([AsF₆]⁻), sulfate ([SO₄]²⁻), tosylate ([C₇H₇SO₃]⁻), nonaflate ([C₄F₉SO₃⁻), tris-(pentafluoroéthyl)-trifluorophosphate ([PF₃(C₂F₅)₃]⁻), thiocyanate ([SCN]⁻), carbonate ([CO₃]²⁻), [R'-COO]⁻, [R'-SO₃]⁻, [R'PO₄R"]⁻ ou [(R'-SO₂)2N]⁻, et R' et R" représentent, en étant identiques ou différents, à chaque fois un radical alkyle aliphatique ou alicyclique linéaire ou ramifié, comprenant 1 à 12 atomes de carbone ou un radical C₅-C₁₈-aryle substitué, (C₅-C₁₈-aryle substitué) -C₁-C₆-alkyle ou C₁-C₆-alkyl- (C₅-C₁₈-aryle substitué), qui peut être substitué par des atomes d'halogène ;
le cation étant choisi parmi :
les cations d'ammonium quaternaire de formule générale [NR¹R²R³R⁴]⁺ ; les cations de phosphonium de formule générale [PR¹R²R³R⁴]⁺; ou les cations d'imidazolium de formule générale 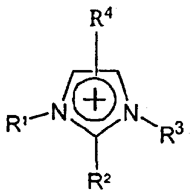 le noyau imidazole pouvant être substitué par au moins un groupe qui est choisi parmi les groupes C₁-C₆-alkyle, C₁-C₆-alcoxy, C₁-C₆-aminoalkyle substitué, C₅-C₁₂-aryle substitué ou (C₅-C₁₂-aryle substitué) -C₁-C₆-alkyle,
les cations de pyridinium de formule générale 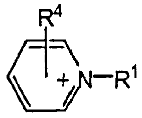 le noyau pyridine pouvant être substitué par au moins un groupe qui est choisi parmi les groupes C₁-C₆-alkyle, C₁-C₆-alcoxy, C₁-C₆-aminoalkyle substitué, C₅-C₁₂-aryle substitué ou (C₅-C₁₂-aryle substitué) -C₁-C₆-alkyle,
les cations de pyrazolium de formule générale 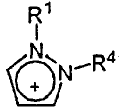 le noyau pyrazole pouvant être substitué par au moins un groupe qui est choisi parmi les groupes C₁-C₆-alkyle, C₁-C₆-alcoxy, C₁-C₆-aminoalkyle substitué, C₅-C₁₂-aryle substitué ou (C₅-C₁₂-aryle substitué) -C₁-C₆-alkyle,
et les cations triazolium de formule générale 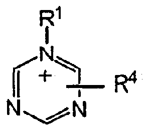 le noyau triazole pouvant être substitué par au moins un groupe qui est choisi parmi les groupes C₁-C₆-alkyle, C₁-C₆-alcoxy, C₁-C₆-aminoalkyle substitué, C₅-C₁₂-aryle substitué ou (C₅-C₁₂-aryle substitué) -C₁-C₆-alkyle,
et les radicaux R¹, R², R³ étant choisis, indépendamment les uns des autres, dans le groupe formé par :
l'hydrogène ;
les groupes alkyle aliphatiques ou alicycliques,
linéaires ou ramifiés, saturés ou insaturés, comprenant 1 à 20 atomes de carbone ;
les groupes hétéroaryle, hétéroaryl-C₁-C₆-alkyle comprenant 3 à 8 atomes de carbone dans le radical hétéroaryle et au moins un hétéroatome choisi parmi N,
O et S, qui peut être substitué par au moins un groupe choisi parmi les groupes C₁-C₆-alkyle et/ou les atomes d'halogène ;
les groupes aryle, aryl-C₁-C₆-alkyle comprenant 5 à 12 atomes de carbone dans le radical aryle, qui peuvent le cas échéant être substitués par au moins un groupe C₁-C₆-alkyle et/ou un atome d'halogène.
et le radical R étant choisi parmi les groupes alkyle aliphatiques ou alicycliques, linéaires ou ramifiés, saturés ou insaturés comprenant 1 à 20 atomes de carbone ; les groupes hétéroaryl-C₁-C₆-alkyle comprenant 4 à 8 atomes de carbone dans le radical aryle et au moins un hétéroatome choisi parmi N, O et S, qui peuvent être substitués par au moins un groupe C₁-C₆-alkyle et/ou un atome d'halogène ; les groupes aryl-C₁-C₆-alkyle comprenant 5 à 12 atomes de carbone dans le radical aryle qui peuvent le cas échéant être substitués par au moins un groupe C₁-C₆-alkyle et/ou un atome d'halogène.

3. Système catalytique selon les revendications 1 à 2, **caractérisé en ce que** la composition catalytiquement active contient du nickel sous une forme soluble pour le liquide ionique utilisé dans les étages d'oxydation de 0 à +2.

4. Système catalytique selon la revendication 3, **caractérisé en ce qu'**on utilise du NiCl₂(P[C₆H₅]3).

5. Système catalytique selon les revendications 1 et 2, **caractérisé en ce qu'**on utilise, comme acide de Lewis présentant des propriétés d'alkylation, des composés organiques de l'aluminium présentant la formule brute générale Al₂XₙR₆₋ₙ avec n = 0-6, X = Cl⁻ ou Br⁻ et R = C₁-C₆-alkyle ou C₅-C₁₂-cycloalkyle ou des mélanges de ces composés.

6. Système catalytique selon les revendications 1 et 2, **caractérisé en ce qu'**on utilise, comme acide de Lewis présentant des propriétés d'alkylation, le dichlorure d'éthylaluminium.

7. Système catalytique selon au moins l'une quelconque des revendications 1 à 6, **caractérisé en ce qu'**on utilise une composition contenant du chlorure de 1-butyl-3-méthylimidazolium et du chlorure d'aluminium.

8. Système catalytique selon au moins l'une quelconque des revendications 1 à 6, **caractérisé en ce qu'**on utilise du tris-(pentafluoroéthyl)-trifluorophosphate de 1-éthyl-3-méthylimidazolium.

9. Système catalytique selon au moins l'une quelconque des revendications 1 à 8, **caractérisé en ce qu'**on utilise, comme matériau support, du dioxyde de silicium présentant une grosseur de particules de 0,063 à 0,2 mm, une surface BET de 250 à 1000 m²/g, déterminée selon les normes DIN 66131 et 66132.

10. Système catalytique selon au moins l'une quelconque des revendications 1 à 9, **caractérisé en ce que** le rapport massique du matériau support, contenant du dioxyde de silicium, au liquide ionique selon la revendication 2 se situe dans une plage de 0,012 à 1,255.

11. Système catalytique selon au moins l'une quelconque des revendications 1 à 9, **caractérisé en ce que** le rapport massique du matériau support, contenant du dioxyde de silicium, qui a été déshydroxylé au préalable de manière connue, au liquide ionique selon la revendication 2 se situe dans une plage de 0,007 à 0,674.

12. Système catalytique selon au moins l'une quelconque des revendications 1 à 9, **caractérisé en ce que** le rapport massique du matériau support, contenant du charbon actif présentant une surface BET entre 1000 m²/g et 4000 m²/g, déterminée selon les normes DIN 66131 et 66132, au liquide ionique selon la revendication 2 se situe dans une plage de 0,022 à 2,171.

13. Procédé d'oligomérisation de mélanges d'hydrocarbures insaturés en utilisant un système catalytique selon au moins l'une quelconque des revendications 1 à 12.

14. Procédé selon la revendication 13, **caractérisé en ce qu'**on utilise, comme mélange d'hydrocarbures insaturés, un flux contenant des oléfines comprenant trois à cinq atomes de carbone.

15. Procédé selon la revendication 14, **caractérisé en ce qu'**on utilise, comme mélange d'hydrocarbures insaturés, un flux contenant des C₄-oléfines linéaires et des hydrocarbures saturés.

16. Procédé selon la revendication 15, **caractérisé en ce qu'**on utilise, comme mélange d'hydrocarbures insaturés, un flux contenant des hydrocarbures saturés à une proportion massique entre 50% et 80% et des C₄-oléfines linéaires.
